# FASCICULE DE BREVET EUROPEEN

(11) **EP 0 879 031 B1**
(45) Date de publication et mention de la délivrance du brevet: **05.11.2003**
(21) Numéro de dépôt: 97904480.7
(22) Date de dépôt: 03.02.1997
(51) Int. Cl.: A61F 2/32

(54) **PROTHESE DE HANCHE NON LUXABLE ET PEU USABLE**
UNLUXIERBARE UND GERING-ABRIEBBARE HÜFTPROTHESE
NON-DISLOCATABLE LOW-WEAR HIP PROSTHESIS

(30) Priorité: 02.02.1996 FR 9601308
(43) Date de publication de la demande: 25.11.1998
(73) Titulaire: VOYDEVILLE, Gilles, F-54000 Nancy (FR)
(72) Inventeur: VOYDEVILLE, Gilles, F-54000 Nancy (FR)
(74) Mandataire: Jacobson, Claude
(86) Numéro de dépôt international: FR9700213
(87) Numéro de publication internationale: WO97027827

(56) Documents cités:
- EP-A- 0 412 438
- EP-A- 0 461 019
- EP-A- 0 639 357
- WO-A-93/03687
- DE-A- 3 200 340
- DE-A- 4 401 815
- DE-U- 9 312 150
- US-A- 4 159 544

## Description

La présente invention a pour objet une prothèse de hanche, comportant une tige fémorale, une tête sphérique adaptée pour coiffer un col de la tige fémorale, un cotyle métallique dans lequel peut s'articuler la tête, et une cupule interposée entre la tête et le cotyle.

comme on le sait, les têtes des tiges fémorales de prothèse de hanche sont généralement articulées sur une pièce en polyéthylène logée dans le cotyle métallique, qui lui-même est impacté dans la paroi osseuse de la cavité cotyloïdienne. La pièce en polyéthylène forme un cotyle interne au cotyle métallique et fixé à ce dernier. Or, les frottements entre la tête métallique ou céramique et la pièce intercalaire en polyéthylène soulèvent certains problèmes. En outre ces prothèses présentent des risques de luxation.

Le problème provoqué par l'apparition de débris de polyéthylène résultant du frottement de la tête de la prothèse contre le cotyle en polyéthylène est bien connu. Cette usure entraîne la formation de micro-débris de polyéthylène, qui eux-mêmes provoquent une réaction macrophagique, laquelle entraîne elle-même la génération d'une membrane d'interposition entre les implants et l'os, cette membrane étant elle-même une cause de descellement.

Le problème de la luxation de hanche est sensiblement différent. Il est connu que les prothèses de hanche comportant une tête de diamètre important, c'est-à-dire d'au moins 32mm, sont peu luxables et relativement stables; les prothèses avec un diamètre plus important, par exemple du type MAC KEE FARRART ou THOMSON, sont encore moins luxables. Par ailleurs on utilise depuis quelque temps des têtes de prothèse de hanche de diamètre notablement inférieur, par exemple 28mm, afin de diminuer l'usure, car le coefficient de friction entre une telle tête et le polyéthylène est moins élevé qu'avec une tête de diamètre plus important.

Toutefois cela n'a pas suffi pour supprimer l'usure, et a entraîné un autre problème qui est celui de l'instabilité des hanches. En effet, le coefficient de friction étant moindre, la hanche est davantage luxable, en particulier dans la période immédiatement consécutive à l'opération.

Par ailleurs, les couples de friction céramique/céramique sont connus et présentent des avantages. Mais ces couples provoquent d'autres difficultés, notamment des risques de descellement et de luxation.

Le document EP-A-0 461 019 décrit une prothèse de hanche selon le préambule de la revendication 1 comportant une tête spherique articulée dans une partie femelle élastique d'une cupule articulée dans un cotyle. La tête peut sortir de la partie femelle élastique, ce qui rend la prothèse luxable.

Enfin on connaît des prothèses comportant une cupule de grand diamètre impactée sur une tête en métal avec interposition de polyéthylène souple pour permettre l'impaction et la rétention sur la tête en métal. Ce système entraîne donc encore une usure métal/polyéthylène. De plus ce type de prothèses provoque des cotyloïdites, car elles sont disposées dans des cotyles osseux, et on observe alors une réaction cotyloïdienne osseuse à l'implant métallique situé en regard.

L'invention a pour but de proposer une prothèse de hanche agencée pour remédier à ces divers inconvénients.

Conformément à l'invention, la prothèse de hanche comprend les caractéristiques mentionnées à la partie caractérisante de la revendication 1

Ainsi la tête, avantageusement en céramique ou matériau équivalent, qui repose sur le col, s'articule dans une cupule également en céramique ou équivalent, comportant suivant une particularité de l'invention des moyens pour la solidariser avec la tête de manière non amovible.

La cupule est avantageusement constituée d'une partie sensiblement hémisphérique et d'un anneau formé par une portion de sphère, fixé à la partie hémisphérique afin d'empêcher celle-ci de se désolidariser de la tête.

Ainsi est réalisée une tête en céramique mobile à l'intérieur d'une seconde tête incomplète en céramique constituée par une cupule que l'on peut qualifier de "rétentive". Pour que cette cupule soit "rétentive", il faut qu'elle s'étende sur plus d'une demi-sphère en y incluant l'anneau complémentaire.

Ce dernier peut être fixé à la partie hémisphérique par tout moyen approprié, notamment collage ou brasure. La brasure peut être faite à l'or, soit à l'usine soit au bloc opératoire, un assemblage par vissage pouvant être également prévu. La méthode la plus fiable semble être une brasure à l'or, réalisée lors de l'usinage.

La prothèse selon l'invention permet l'obtention d'un frottement très faible, notamment céramique/céramique, produisant très peu de produits de dégradation, grâce à un coefficient de friction extrêmement bas, et elle est donc peu usable. En effet le couple céramique/céramique est celui qui jusqu'à présent présente le plus faible coefficient de friction connu.

En outre, la cupule en céramique est avantageusement non désolidarisable de la tête en céramique, car la brasure est exécutée de manière solide en usine.

Par ailleurs la tête double constituée par la tête en céramique et la cupule dans laquelle elle est articulée présente un diamètre extérieur élevé, qui a l'avantage d'être peu luxable.

Cet agencement reste soumis à une usure, mais du fait que l'articulation a toujours tendance à s'effectuer là où existe le moindre coefficient de friction, l'invention permet de réaliser la principale articulation entre la tête et la cupule associée, dans laquelle elle est emprisonnée. En effet la seconde articulation entre la cupule et le cotyle en polyéthylène n'intervient que lors d'amplitudes extrêmes, donc rarement. Corrélativement, la rareté de ces articulations génère d'autant moins de débris de polyéthylène et donc diminue considérablement les risques de descellements par réaction macrophagique.

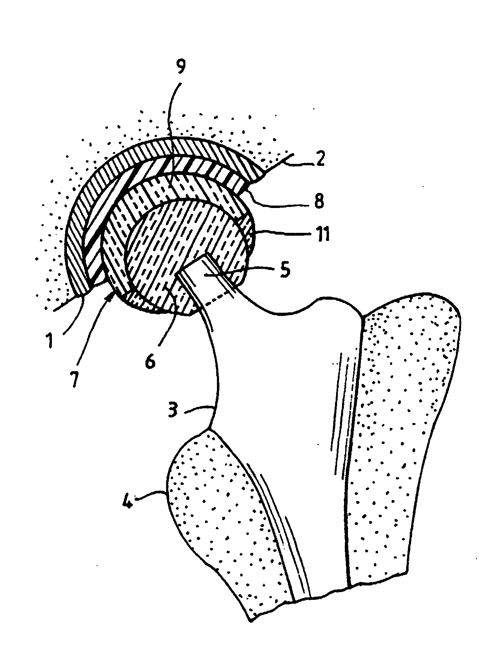

D'autres particularités et avantages de l'invention apparaîtront au cours de la description qui va suivre, faite en référence au dessin annexé qui en illustre une forme de réalisation à titre d'exemple non limitatif.

La figure unique est une vue mi-coupe mi-élévation d'un mode de réalisation de la prothèse de hanche conforme à l'invention.

La prothèse de hanche représentée à la figure unique comprend un cotyle 1 pouvant être ancré par impaction dans la cavité cotyloïdienne de l'os iliaque 2 d'un patient, une tige fémorale 3 pouvant être ancrée à l'extrémité supérieure d'un fémur 4, la tige 3 étant terminée par un col 5.

La prothèse comprend également une tête sphérique 6 dans laquelle est ménagé un alésage convenablement dimensionné pour permettre à la tête 6 de coiffer le col 5, et une cupule 7 interposée entre la tête 6 et un cotyle 8 en matière plastique, de préférence en polyéthylène. Ce cotyle 8 est fixé sur la paroi intérieure du cotyle 1, lequel est métallique, de préférence en titane.

La cupule 7 comporte des moyens pour la solidariser avec la tête 6 de manière non amovible. Dans le mode de réalisation représenté, la cupule 7 est ainsi constituée d'une partie sensiblement hémisphérique 9, adaptée pour recevoir la tête sphérique correspondante 6, et d'un anneau 11 formé par une portion de sphère. L'anneau 11 est fixé à la partie hémisphérique 9 par tout moyen approprié, par exemple vissage ou collage ou brasure, notamment à l'or comme précédemment indiqué. Cet assemblage est exécuté après introduction préalable de la tête 6 dans la partie hémisphérique 9.

La cupule 7 ainsi réalisée occupe donc un volume supérieur à une demi-sphère, de sorte qu'elle ne peut plus être désolidarisée de la tête 6, laquelle reste articulée librement à l'intérieur de la cupule 7. A titre d'exemples, l'anneau 11 peut être formé d'une portion d'environ 1/4 ou 1/6 ou 1/8 de sphère environ.

La tête 6 et la cupule 7 sont avantageusement réalisées en céramique ou autre matériau possédant un très faible coefficient de friction par exemple un composé métallique à très faible coefficient de friction, avec implantation ionique. La prothèse ainsi constituée possède donc une double articulation, à savoir une première articulation entre la tête 6 et la cupule 7, et une seconde articulation entre la cupule 7 et le cotyle 8 en matière plastique, ce qui présente les avantages exposés précédemment.

A titre d'exemple numérique non limitatif, la tête 6 peut présenter un diamètre de 22, 26 ou 28mm, le diamètre maximum approprié semblant être 28mm pour des raisons d'encombrement. L'épaisseur de la cupule 7 peut être d'environ 5mm, de sorte que son diamètre est de 22,26 ou 28mm + (5x2) soit 32,36 ou 38mm. Ainsi est réalisée une tête double d'articulation dont le diamètre extérieur est peu luxable.

Le cotyle en polyéthylène 8 a de préférence une épaisseur minimale de 6mm, le cotyle 1, de préférence en titane, ayant une épaisseur de 1 ou 2mm seulement, afin d'être peu encombrant.

Finalement, la prothèse atteint un diamètre extérieur (cotyle 1), de 50mm de diamètre, ce qui peut être réalisé dans pratiquement 98% des cas.

Pour les 2% restants, la prothèse peut comporter une tête 6 d'un diamètre de 22mm seulement, qui, ajouté à 5x2mm de diamètre supplémentaire pour la cupule 7 donne à celle-ci un diamètre extérieur de 32mm. En y ajoutant 6mm d'épaisseur pour le cotyle 8 on parvient à 44mm, puis avec 2mm d'épaisseur au total pour le cotyle 1, on parvient à une prothèse d'un diamètre total extérieur de 46mm.

La double articulation est donc composée d'une articulation "rétentive" non luxable et d'une articulation non rétentive avec le cotyle en polyéthylène; ainsi est obtenue une prothèse moins luxable que les prothèses classiques de 22, 26, 28 mm de diamètre.

La prothèse selon l'invention comporte donc une tête 6 non désolidarisable de la cupule 7 sur laquelle elle est articulée, et dont les mouvements ne produisent aucun débris ou aucune dégradation en raison des matériaux céramiques ou équivalents utilisés. La seconde articulation entre la cupule 7 et le cotyle 8 fonctionne très peu grâce à l'articulation précédente qui présente un coefficient de friction inférieur à celui de la cupule et du cotyle 8. De ce fait les débris produits sur cette dernière articulation sont si faibles qu'ils permettent de porter la durée de vie effective de la prothèse de 15 ans à 20 ou 25 ans.

## Revendications

1. Prothèse de hanche comportant une tige fémorale (3), une tête sphérique (6) adaptée pour coiffer un col (5) de la tige fémorale, un cotyle métallique (1) dans lequel peut s'articuler la tête et une cupule (7) interposée entre la tête (6) et le cotyle, ladite tête et la cupule étant en un matériau à faible coefficient de frottement, et la tête étant articulée sur la cupule, la cupule étant elle-même articulée dans un cotyle (8) en matière plastique fixé dans le cotyle métallique (1), et ledit matériau à faible coefficient de frottement étant une céramique ou autre matériau possédant un très faible coefficient de friction, par exemple un composé métallique à très faible coefficient de friction avec implantation ionique, **caractérisée en ce que** la cupule (7) comporte des moyens pour la solidariser avec la tête (6) de manière non amovible.

2. Prothèse selon la revendication 1, **caractérisée en ce que** la cupule (7) est constituée d'une partie sensiblement hémisphérique (9) et d'un anneau (11) formé par une portion de sphère, fixé à la partie hémisphérique afin d'empêcher celle-ci de se désolidariser de la tête.

3. Prothèse selon la revendication 2, **caractérisée en ce que** l'anneau( 11) est fixé à la partie hémisphérique (9) par collage ou brasure ou autre moyen approprié.

4. Prothèse selon l'une des revendications 2 et 3, **caractérisée en ce que** l'anneau (11) constitue une portion d'environ un quart ou un sixième ou un huitième de sphère.

5. Prothèse selon l'une des revendications 1 à 4 **caractérisée en ce que** le cotyle (8) de matière plastique est en polyéthylène et le cotyle métallique (1) est en titane.

## Patentansprüche

1. Hüftprothese, bestehend aus einem Femoralschaft (3), einem Kugelkopf (6), welcher zum Aufbringen auf einen Hals (5) des Femoralschaftes ausgebildet ist, einer metallischen Gelenkpfanne (1), in welcher der Kugelkopf und eine zwischen dem Kugelkopf (6) und der Gelenkpfanne angeordnete Kappe (7) verschwenkbar sind, wobei der Kugelkopf und die Kappe aus einem Werkstoff mit einer kleinen Reibungszahl bestehen, der Kugelkopf in der Kappe verschwenkbar ist, und die Kappe selbst in einer aus Kunststoff bestehenden und in der metallischen Gelenkpfanne (1) befestigten Gelenkpfanne (8) verschwenkbar gelagert ist, und wobei der Werkstoff mit einer kleinen Reibungszahl aus einem keramischen oder anderem Material besteht, welches einen sehr geringen Reibungskoeffizienten aufweist, zum Beispiel eine metallische Zusammensetzung mit Ionenimplantation mit einem sehr geringen Reibungskoeffizienten, **dadurch gekennzeichnet, dass** die Kappe (7) Mittel umfasst, welche eine unbewegliche Festlegung mit dem Kugelkopf (6) ermöglichen.

2. Hüftprothese nach Anspruch 1, **dadurch gekennzeichnet, dass** die Kappe (7) aus einem im Wesentlichen halbkugelförmigen Teil (9) und einem aus einem Teil einer Kugel gebildeten Ring (11) besteht, welcher an dem halbkugelförmigen Teil befestigt ist, um ein Loslösen dieses Teils von dem Kugelkopf zu verhindern.

3. Hüftprothese nach Anspruch 2, **dadurch gekennzeichnet, dass** der Ring (11) an dem halbkugelförmigen Teil (9) mittels einer Klebverbindung, einer Hartlötung oder einer anderen geeigneten Verbindungsart befestigt ist.

4. Hüftprothese nach einem der Ansprüche 2 und 3, **dadurch gekennzeichnet, dass** der Ring (11) ungefähr ein Viertel oder ein Sechstel oder ein Achtel eines Teils einer Kugel umfasst.

5. Hüftprothese nach einem oder mehreren der Ansprüche 1 bis 4, **dadurch gekennzeichnet, dass** die Gelenkpfanne (8) aus Kunststoff aus Polyäthylen und die metallische Gelenkpfanne (1) aus Titan besteht.

## Claims

1. Hip prosthesis comprising a femoral stem (3), a spherical head (6) suitable for being placed over a neck (5) of the femoral stem, a metal cotyle (1) in which the head may be articulated, and a cup (7) interposed between the head (6) and the cotyle, the head and the cup being composed of a material having a low coefficient of friction, and the head being articulated on the cup, the cup being itself articulated in a cotyle (8) which is composed of plastics material and which is secured in the metal cotyle (1), and the material having a low coefficient of friction being a ceramic or another material having a very low coefficient of friction, for example a metal compound having a very low coefficient of friction with ion implantation, **characterised in that** the cup (7) comprises means for joining it securely to the head (6) in a non-removable manner.

2. Prosthesis according to claim 1, **characterised in that** the cup (7) is constituted by a substantially hemispherical portion (9) and by a ring (11) which is formed by a portion of a sphere and which is secured to the hemispherical portion in order to prevent the latter from becoming detached from the head.

3. Prosthesis according to claim 2, **characterised in that** the ring (11) is secured to the hemispherical portion (9) by adhesive bonding or brazing or another appropriate means.

4. Prosthesis according to either claim 2 or claim 3, **characterised in that** the ring (11) constitutes a portion of approximately one quarter or one sixth or one eighth of a sphere.

5. Prosthesis according to any one of claims 1 to 4, **characterised in that** the cotyle (8) of plastics material is composed of polyethylene and the metal cotyle (1) is composed of titanium.
